Europäisches Patentamt
European Patent Office
Office européen des brevets

(19)

(11) Publication number: **0 213 814 A2**

(12) # EUROPEAN PATENT APPLICATION

(21) Application number: 86306135.4

(22) Date of filing: 08.08.86

(51) Int. Cl.⁴: C 07 D 513/04 //A01N43/90, (C07D513/04, 249:00, 277:00)

(30) Priority: 09.08.85 US 764026

(43) Date of publication of application: 11.03.87 Bulletin 87/11

(84) Designated Contracting States: AT BE CH DE FR GB IT LI NL SE

(71) Applicant: ELI LILLY AND COMPANY
Lilly Corporate Center
Indianapolis Indiana 46285(US)

(71) Applicant: ELANCO/IHARA K.K.
582 Shio Shinden Fukude-cho
Iwata-Gun Shizuoka-Ken(JP)

(71) Applicant: IHARA CHEMICAL INDUSTRY CO., LTD.
4-26, Ikenohata 1-chome
Taitoh-ku, Tokyo(JP)

(72) Inventor: Achgill, Raplh Kenneth
7031 East 200 South
Lafayette Indiana 47905(US)

(72) Inventor: Matsuura, Norio
279-3 Aza-Umenoki Higashihara
Iwata-Gun Shizuoka-Ken(JP)

(72) Inventor: Tabuchi, Fumiya
3112-1, Nakanogou Fujikawa-Cho
Ihara-Gun Shizuoka-Ken(JP)

(74) Representative: Tapping, Kenneth George et al,
Erl Wood Manor
Windlesham Surrey, GU20 6PH(GB)

(54) **Synthesis of tricyclazole.**

(57) New and advantageous one-step process for preparing tricyclazole in high yield free from impurities.

EP 0 213 814 A2

Croydon Printing Company Ltd

# SYNTHESIS OF TRICYCLAZOLE

This invention provides a new and particularly efficient process for preparing 5-methyl-1,2,4-triazolo[3,4-b]benzothiazole, a well-known systemic fungicide for the control of Piricularia oryzae, the causative organism of rice blast. The product of this invention is known by the generic name "tricyclazole," and that name will be used hereinafter. Tricyclazole is described and claimed in United States Patent No. 4,064,261, issued 20 September, 1977, in Japanese Patent No. 984,228, issued 22 January, 1980, in Korean Patent No. 5,132, issued 7 August, 1976, and in Taiwanese Patent No. N19543, issued 13 October, 1977.

Those patents illustrate various processes for preparing tricyclazole, which processes are not as advantageous as is the present process.

This invention provides a process for preparing tricyclazole of the formula

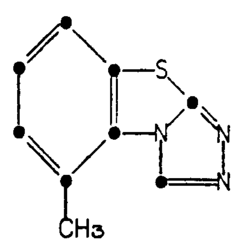

characterized by reacting 2-amino-4-methylbenzothiazole of the formula

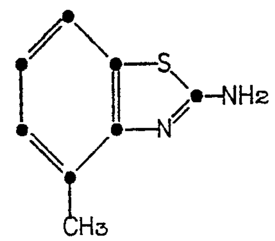

with hydrazine or its hydrate in the presence of ethylene glycol and hydrochloric acid at from about 100° to about 150° to form 2-hydrazino-4-methylbenzothiazole of the formula

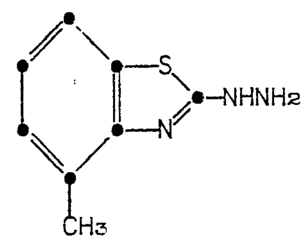

cooling the mixture below about 100°, and adding xylene; continuing to cool the mixture to from about 10° to about 45°, and allowing the layers to separate; removing the ethylene glycol layer; adding water; allowing the mixture to separate; removing the water layer; adding to the xylene layer an amount of irreversible acid scavenger which is slightly in excess of the amount necessary to neutralize the mixture; distilling the neutralized xylene layer at liquid temperatures from about 100° to about 120° to remove an azeotrope of water

and xylene; continuing to distill the mixture to remove an azeotrope of ethylene glycol and xylene, until the ethylene glycol is substantially all removed; cooling the mixture below about 100°, if necessary; adding to the mixture at least about 5.0 moles per mole of 2-hydrazino-4-methylbenzothiazole, of formic acid of at least about 80% by weight concentration; and holding the mixture at an elevated temperature until the desired product is formed. The product is preferably recovered by distilling the excess formic acid from the mixture; adding additional xylene if needed to provide a flowable slurry, combining the mixture with sufficient water to dissolve inorganic impurities and separating the crystalline material.

All temperatures recited herein are in degrees centigrade.

The above process is efficient and has been found to produce tricyclazole in high yields. The present process provides the product consistently in high purity without the need for additional expensive purification steps, so that it may be used for agricultural purposes without fear of injury to the crop. Further, a major advantage of the present process is the ability to prepare tricyclazole in one-step from 2-amino-4-methylbenzothiazole.

In the first step of the process, 2-amino-4-methylbenzothiazole (AMBT) is reacted with hydrazine, or hydrazine hydrate, in the presence of hydrochloric acid (preferably concentrated) and ethylene glycol. It is preferred that no other organic solvent be present in the first step. However, when recycled ethylene glycol

is employed, from approximately 5% to 10% of xylene (v/v) will also be present. The concentration of AMBT is of some importance and should be in the range of approximately 1 gram mole per 500 ml. of ethylene glycol. The concentration may of course be varied, in the range of from about 300 ml. to about 750 ml. per gram mole, but optimal results are obtained when the concentration is close to 500 ml. of solvent per gram mole.

It is preferred for the reaction mixture to contain at least about 1 mole of hydrochloric acid per mole of AMBT, although large excess amounts of acid seem to serve no purpose. Small excess amounts of acid in the usual range of from about a few percent excess to about 50% excess may be used at the discretion of the operator. As will be shown below, the ethylene glycol solvent can and should be recycled, and some hydrochloric acid will be recycled along with the solvent. The operator should analyze the recycled ethylene glycol and take into account the amount of acid remaining in it when calculating the next batch.

It is preferred to use at least about 3 moles of hydrazine per mole of AMBT. The exact amount of hydrazine is not critical, but best results will not be obtained with less than about 3 moles of it in the reaction mixture. Larger amounts, up to about 5 moles, do no harm but also do not appear to confer any particular benefit. Use of amounts of hydrazine in excess of about 5 moles is not recommended. Again, the amount of hydrazine in the recycled ethylene glycol should be

measured and taken into account when a recycle process is used. Hydrazine may be used in the pure form, or as a hydrate, as may be economical and convenient in a given case.

When the first-step reaction mixture has been prepared, it is heated to an elevated temperature from about 100° to about 150°. It will be understood that such an operation requires equipment capable of condensing the organic vapors which leave the mixture and returning the condensate to the reaction vessel. It is preferable that the reaction vessel be equipped for operation as a distillation vessel, also, to avoid transferring the reaction mixture from one vessel to another in the later steps. It is preferred to react the AMBT with hydrazine at a temperature from about 120° to about 140°, most preferably at a temperature from about 125° to about 130°. It will often be necessary to distill off some small amount of water to raise the boiling point of the mixture to the desired operating temperature, especially when the batch contains recycled ethylene glycol or when dilute solutions of hydrazine hydrate are used. The necessary reaction time for efficient conversion of AMBT to the hydrazino intermediate (called HBT) is in the range of about 1 to 4 hours. It will be understood that the optimum time must be determined by the operator in each case, and depends upon the operating temperature, and also upon the relative importance in the given case of high yield of product, as compared to quick throughput of product through the equipment.

When the reaction has gone as far as is desired, the mixture is cooled. The exact temperature is not important, but it should be cooled below about 100°, and then xylene, in the general range of about 400 ml. per gram-mole of AMBT, is added. The exact amount of xylene is not critical, but it is preferred to use an amount in the general range of from about 300 to about 500 ml. per gram-mole, and an amount which also is preferably equal in volume to about 40 to 80%, especially about 60 to 70%, of the volume of the ethylene glycol.

The HBT in the mixture starts to precipitate as soon as the xylene is added. The mixture is cooled to from about 10° to about 45° while the HBT precipitates, most preferably to about 20-25°, and still more preferably to the ambient temperature. When the mixture is cooled, agitation is stopped, or is reduced to a very mild level, and the ethylene glycol and xylene layers are allowed to separate. The precipitate of HBT goes to the upper, xylene layer.

When the layers have separated, the ethylene glycol layer is removed and is discarded or, preferably, held to be recycled in a later batch. The HBT-containing xylene layer is then washed with water, and the water layer is separated. The HBT-containing xylene layer is then neutralized by the addition of a base which acts as an irreversible acid scavenger and forms a salt which is stable to high temperature. Exemplary bases of this type are alkali metal hydroxides, bicarbonates or carbonates. Sodium, potassium and

lithium are included in the term "alkali metal", of which sodium and potassium, especially sodium, are preferred. The carbonates are preferred. An amount of the base is added which is somewhat in excess of the amount necessary to neutralize the residual hydrochloric acid in the xylene layer.

The neutralized mixture is then distilled to remove water and ethylene glycol, as well as some of the starting compounds. As the mixture is heated to about 100-110°, an azeotrope of water and xylene begins to boil from it. It is most convenient, although by no means essential, to pass the condensate from the azeotropic distillation through a decanter, and to return the separated xylene to the reaction mixture. The xylene-water distillation is continued until the water is essentially gone, at a temperature about 120°. Excess hydrazine will also distill off near this temperature.

One of the particularly advantageous aspects of the present invention is that ethylene glycol can be removed by means of an ethylene glycol-xylene azeotrope, which begins to distill from the mixture after the water has been distilled away. That azeotrope is removed at temperatures between about 75° and about 100° when carried out under reduced pressure, which is preferred, or at temperatures between about 136° and about 143° when carried out under standard pressure, which temperature is reached when substantially all of the ethylene glycol has been removed from the mixture, leaving the

HBT in the form of a suspension in practically pure xylene.

The distillate from the ethylene glycol-xylene azeotrope can be decanted, and it is preferred to do so and return the xylene to the mixture, while saving the recovered ethylene glycol for reuse in a future process.

Those skilled in the art will understand that the boiling points given for xylene herein are variable, because "xylene" is actually a mixture of the three possible isomers, ortho, meta and para-xylene. Accordingly, the boiling point of commercial xylene varies over a small range, depending on the relative amount of the three isomers which it contains. Commercial xylene is preferably employed in the present process and, in addition to containing the three xylene isomers, will also contain ethylbenzene.

When substantially all of the ethylene glycol has been removed from the xylene-HBT suspension, the temperature of the mixture is cooled, if necessary, below about 100° without isolating the HBT.

To the xylene-HBT slurry is then added an excess amount of formic acid. Preferably, at least about 5.0 moles of formic acid are used for each mole of HBT, but 10.5 moles of formic acid have also provided excellent results. It has been found that amounts of formic acid greater than 10.5 moles are not harmful to the process in any way, but neither are larger amounts beneficial, and there is therefore no reason to use amounts in excess of about 10.5 moles. It is important, however, that the concentration of the formic acid be at

least about 80% by weight, preferably about 85% to about 95% by weight. Higher concentrations of formic acid are preferred when using a lesser number of moles of formic acid. Accordingly, when recycled formic acid is used, it is important to check its concentration analytically, and it may be necessary to concentrate recycled formic acid by distillation or add purer formic acid in order to obtain the necessary high concentration.

The mixture is then heated, and is held with appropriate stirring at an elevated temperature until the HBT has been converted to tricyclazole. The preferred and most convenient temperature is the ambient pressure reflux temperature, about 100-110°. The reaction temperature is not critical, however, but may be any convenient elevated temperature in the approximate range of from about 50° to about 150°. Of course, pressure above atmospheric may be used as necessary to raise the boiling point of the mixture. The preferred reaction time is in the range of about 4 hours, but the operator of the process can adjust the reaction time to suit his goals, as described above.

As the tricyclazole is formed, it dissolves in the formic acid layer in the reaction vessel, and the xylene is accordingly substantially free of product when the reaction is complete. In instances where a lesser number of moles of formic acid are used for each mole of HBT, it is preferred to proceed with the reaction while removing xylene by azeotropic distillation. The xylene layer may be removed from the reaction vessel by layer separation, or it may be left in the reaction vessel

during the following steps. In either case, excess formic acid is removed by distillation. The contents of the vessel are heated, if at ambient pressure, to about 100-110° to start the distillation of formic acid. If the xylene has been left in the vessel, the entire amount of excess formic acid can be removed by azeotropic distillation. The final liquid temperature, when substantially all of the formic acid has been removed, is about 139° to 141°, depending upon the mixture of isomers in the xylene. Of course, the distillation can be conducted under a partial vacuum at correspondingly lower temperatures.

On the other hand, if the xylene layer was removed from the reaction vessel before starting the distillation, only about 80% (v/v) of the excess formic acid can be removed by distillation. In this case, the distillation should be stopped when a liquid temperature of about 130° is reached (at ambient pressure), and the contents of the vessel should be cooled somewhat and additional xylene added. An amount in the range of about 300-400 ml. of xylene per gram-mole of product is convenient. The mixture is reheated and azeotropic distillation is carried out to remove substantially all of the remaining formic acid, to a final liquid temperature of about 139° to about 141°, at ambient pressure.

At this point, the contents of the reaction vessel consists of a solution of tricyclazole in xylene, containing relatively small amounts of organic and inorganic impurities. It may be advisable to add some additional xylene as the mixture is cooled, to assure

that the suspension can be conveniently pumped and filtered.

The suspension is cooled somewhat and combined with water in order to dissolve inorganic impurities, typically by adding the suspension to water. Approximately 100 ml. to 200 ml. of water per gram-mole of starting AMBT is adequate.

The mixture is cooled down to approximately the ambient temperature, for the sake of convenience, and the product is collected on a suitable filter or centrifuge. Finally, the filter is washed with additional water and xylene to achieve the desired purity, and the washed solids are dried. Any type of drying equipment can be used, so long as the temperature of the solids is not elevated substantially above about 100-110°.

Typically, when the process is operated according to the preferred conditions discussed above, the yield will be in the range of about 85%-93% of the theoretical yield, based on the amount of AMBT put into the process, and the product will be about 98% pure. Further, it will be found that the ethylene glycol, the xylene, most of the excess hydrazine, and most of the hydrochloric acid put into the process can be recycled, and that some of the excess formic acid recovered from the process by distillation can be reconstituted with 98% commercial formic acid to give fresh 80% formic acid to be used in future lots.

The present invention is further illustrated and explained by the following Examples.

## Example 1

To a suitable glass-lined reactor, equipped with a heating and cooling jacket, an agitator, and a condenser, decanter and condensate return and collection valves, was charged 8000 l. of recycled ethylene glycol (containing 873 kg. of hydrazine monohydrate and 355 kg. of hydrochloric acid), 1400 kg. of AMBT, and 460 kg. of hydrazine monohydrate (100%). No additional hydrochloric acid was required.

The mixture was heated with agitation to 125-130°, and was stirred at constant temperature for 2 hours. The mixture was then cooled to less than 95°, 2900 l. of xylene was added, and the suspension was cooled to 30-35°. The agitator was stopped, the layers were allowed to separate, and the lower ethylene glycol layer was removed to be recycled to a subsequent lot.

To the xylene-HBT slurry was added 2000 l. of water, and the suspension was stirred for 15 minutes. The agitation was stopped, the layers were allowed to separate, and the lower water layer was removed.

To the xylene-HBT slurry was added 10 kg. of sodium carbonate, and the suspension was then heated to the boiling point with agitation, and the vapor was condensed and passed through a decanter. The xylene-water azeotrope was removed, and the xylene layer of the condensate was returned to the vessel. The azeotropic distillation was continued until the liquid temperature of the mixture was 110°, at which time substantially all of the water had been removed from the mixture. The

xylene-ethylene glycol azeotrope began distilling under reduced pressure, and the liquid temperature of the mixture was allowed to cool to 85° and was maintained at that temperature. The condensate from that azeotrope was also separated in the decanter, and the xylene was returned to the vessel while the ethylene glycol was held for reuse. The azeotrope was continued until substantially all of the ethylene glycol was removed. The slurry was then cooled to 60-65°. At this point a second slurry, run substantially the same as the above, was combined with the above slurry and further processing was continued.

To the combined slurry was added, with agitation, 3230 kg. of 93.7% aqueous formic acid and 1180 kg. of 76% aqueous formic acid recovered from a previous lot. The contents of the vessel were heated to the boiling point, and the vapor was condensed and passed through a decanter. The xylene layer was removed and held for reuse later in the process, and the formic acid layer was returned to the vessel. The azeotropic distillation was continued until substantially all the xylene was removed. The vessel contents were stirred under reflux, about 110°, for 2 hours. Distillation was then started, collecting the condensate in a suitable vessel. Distillation was continued, observing the liquid temperature in the vessel, until the liquid temperature reached 130°, at which time the vessel contents were cooled to less than 110° and the previously held xylene and 150 l. of fresh xylene were added. The mixture was then heated to boiling and the vapor was

condensed and passed through a decanter. The xylene-formic acid azeotrope was held for reuse in the next lot, and the xylene layer was returned to the vessel. Distillation was continued until the liquid temperature reached 142°, at which time the condensate was substantially pure xylene. At this point 2065 l. of xylene was added and the mixture was agitated for 1 hour. The mixture was cooled to 30°, and transfered to an agitated vessel containing 8000 l. of water, and stirred for 15 mintues.

The slurry was filtered through a Basket centrifuge to collect the solid product. The product was washed on the centrifuge with a small amount of water and xylene, and the washed product was collected and dried in a cone dryer, and heated under vacuum to 70° until substantially all of the xylene had been removed.

The product was 3014 kg. of tricyclazole (93.1% yield), as described in the patents mentioned at the beginning of this document, which was found to be 98.7% pure by liquid chromatogaphic analysis.

## Example 2

To a suitable glass-lined reactor, equipped with a heating and cooling jacket, an agitator, and a condenser, decanter and condensate return and collection valves, was charged 8000 l. of recycled ethylene glycol (containing 864 kg. of hydrazine monohydrate and 355 kg. of hydrochloric acid), 1400 kg. of AMBT, and 470 kg. of

hydrazine monohydrate (100%). No additional hydrochloric acid was required.

The mixture was heated with agitation to 125-130°, and was stirred at constant temperature for 2 hours. The mixture was then cooled to less than 95°, 2900 l. of xylene was added, and the suspension was cooled to 30-35°. The agitator was stopped, the layers were allowed to separate, and the lower ethylene glycol layer was removed to be recycled to a subsequent lot.

To the xylene-HBT slurry was added 2000 l. of water, and the suspension was stirred for 15 minutes. The agitation was stopped, the layers were allowed to separate, and the lower water layer was removed.

To the xylene-HBT slurry was added 10 kg. of sodium carbonate, and the suspension was then heated to the boiling point with agitation, and the vapor was condensed and passed through a decanter. The xylene-water azeotrope was removed, and the xylene layer of the condensate was returned to the vessel. The azeotropic distillation was continued until the liquid temperature of the mixture was 110°, at which time substantially all of the water had been removed from the mixture. The distillation was continued at standard pressure in order to remove an azeotrope of ethylene glycol and xylene, until the ethylene glycol was substantially all removed. The slurry was then cooled to 65-70°, and with agitation 2184 kg. of 95% aqueous formic acid and 3092 kg. of 69.7% aqueous formic acid recovered from previous lot was added. The contents of the vessel were heated to the boiling point, about 104°, and stirred under reflux

for 4 hours. The mixture was then cooled to 55-60°, and the agitator was stopped, the layers were allowed to separate and the lower formic acid layer was removed for further processing. The xylene layer was held for reuse later in the process. At this point a second formic acid layer, run substantially the same as the above, was combined with the above formic acid and further processing continued. The mixture was then heated to boiling, and the vapor was condensed and collected in suitable vessels. The first 3600 kg. of formic acid distillate was discarded and the liquid temperature was allowed to reach 130°. The remaining formic acid distillate was saved for reuse. The liquid was then cooled to 110°, and the previously held xylene and 150 l. of fresh xylene were added. The mixture was then heated to boiling and the vapor was condensed and passed through a decanter. The xylene-formic acid azeotrope was held for reuse in the next lot, and the xylene layer was returned to the vessel.

Distillation was continued until the liquid temperature reached 142°, at which time the condensate was substantially pure xylene. At this point 2065 l. of xylene was added and the mixture was agitated for 1 hour. The mixture was cooled to 30°, and transferred to an agitated vessel containing 8000 l. of water, and stirred for 15 minutes.

The slurry was filtered through a basket centrifuge to collect the solid product. The product was washed on the centrifuge with a small amount of water and xylene, and the washed product was collected

and dried in a cone dryer, and heated under vacuum to 70° until substantially all of the xylene had been removed.

The product was 2954 kg. of tricyclazole (91.5% yield), as described in the patents mentioned at the beginning of this document, which was found to be 98.9% pure by liquid chromatographic analysis.

CLAIMS

1. A process for preparing tricyclazole of the formula

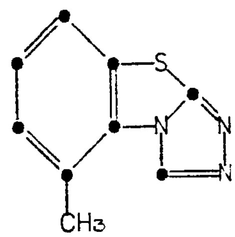

characterized by reacting 2-amino-4-methylbenzothiazole of the formula

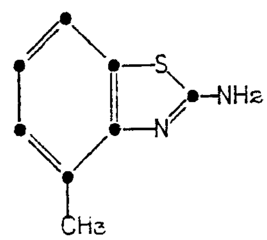

with hydrazine or its hydrate in the presence of ethylene glycol and hydrochloric acid at from about 100° to about 150° to form 2-hydrazino-4-methylbenzothiazole of the formula

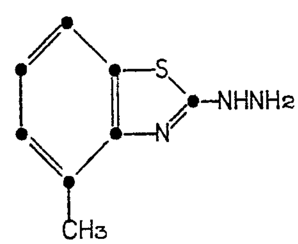

cooling the mixture below about 100°, and adding xylene; continuing to cool the mixture to from about 10° to about 45°, and allowing the layers to separate; removing the ethylene glycol layer; adding water; allowing the mixture to separate; removing the water layer; adding to the xylene layer an amount of irreversible acid scavenger which is slightly in excess of the amount necessary to neutralize the mixture; distilling the neutralized xylene layer at liquid temperatures from about 100° to about 120° to remove an azeotrope of water and xylene; continuing to distill the mixture to remove an azeotrope of ethylene glycol and xylene, until the ethylene glycol is substantially all removed; cooling the mixture below about 100°, if necessary; adding to the mixture at least about 5.0 moles per mole of 2-hydrazino-4-methylbenzothiazole, of formic acid of at least about 80% by weight concentration and holding the mixture at an elevated temperature until the desired product is formed.

2. A process according to Claim 1 wherein the tricyclazole is recovered by distilling the excess formic acid from the mixture; adding additional xylene if needed to provide a flowable slurry; combining the mixture with sufficient water to dissolve inorganic impurities; cooling the mixture, and separating the crystalline product.

3. A process according to Claim 1 or 2 characterized by adding xylene in an amount which is equal in volume to about 40% to about 80% of the volume of the ethylene glycol.

4. A process according to Claim 3 characterized by adding xylene in an amount which is equal in volume to about 60% to about 70% of the volume of the ethylene glycol.

5. A process according to any one of Claims 1 to 4, wherein the irreversible acid scavenger is a carbonate.

6. A process according to any one of the preceding claims, wherein the formic acid has a concentration of about 85% by weight.

7. A process according to any one of the preceding claims, wherein 5.0 moles of formic acid are employed for each mole of 2-hydrazino-4-methylbenzothiazole.

8. A process according to any one of the preceding claims, wherein 10.5 moles of formic acid are employed for each mole of 2-hydrazino-4-methylbenzothiazole.